(19) Europäisches Patentamt
European Patent Office
Office européen des brevets

(11) EP 0 916 963 B1

(12) EUROPEAN PATENT SPECIFICATION

(45) Date of publication and mention of the grant of the patent:
**28.07.1999 Bulletin 1999/30**

(51) Int Cl.[6]: **G01R 33/46**

(21) Application number: **97118127.6**

(22) Date of filing: **18.10.1997**

# (54) Transverse relaxation-optimized NMR spectroscopy (TROSY)

NMR-Spektrokopie mit optimierter transversaler Relaxationszeit (TROSY)

Spectroscopie RMN à temps de relaxation transversale optimisé (TROSY)

(84) Designated Contracting States:
**CH DE FR GB LI**
Designated Extension States:
**AL LT LV RO SI**

(43) Date of publication of application:
**19.05.1999 Bulletin 1999/20**

(73) Proprietor: **Bruker AG**
**8117 Fällanden (CH)**

(72) Inventors:
- **Pervushin, Konstantin, Dr.**
  **8057 Zürich (CH)**
- **Wider, Gerhard, Dr.**
  **8704 Herrliberg (CH)**
- **Riek, Roland**
  **6432 Rickenbach (CH)**
- **Wüthrich, Kurt, Prof. Dr.**
  **8304 Wallisellen (CH)**

(74) Representative: **KOHLER SCHMID + PARTNER**
**Patentanwälte**
**Ruppmannstrasse 27**
**70565 Stuttgart (DE)**

(56) References cited:
- **G.WAGNER: "Prospects for NMR of large proteins" JOURNAL OF BIOMOLECULAR NMR, vol. 3, 1993, pages 375-385, XP002051025**
- **L.E.KAY ET AL.: "Pulse Sequences for Removal of the Effects of Cross Correlation between Dipolar and Chemical-Shift Anisotropy Relaxation Mechanisms on the Measurement of Heteronuclear T1 and T2 Values in Proteins" JOURNAL OF MAGNETIC RESONANCE, vol. 97, 1992, pages 359-375, XP002051026**
- **J.BOYD ET AL.: "Influence of cross-correlation between dipolar and chemical shift anisotropy relaxation mechanisms upon the transverse relaxation rates of 15-N in macromolecules" CHEMICAL PHYSICS LETTERS, vol. 187, no. 3, 6 December 1991, pages 317-324, XP002051027**
- **S.WIMPERIS ET AL.: "Relaxation-allowed cross-peaks in two-dimensional N.M.R. correlation spectroscopy" MOLECULAR PHYSICS, vol. 66, no. 5, 1989, pages 897-919, XP002051028**
- **G.BODENHAUSEN ET AL.: "Natural Abundance Nitrogen-15 NMR by Enhanced Heteronuclear Spectroscopy" CHEMICAL PHYSICS LETTERS, vol. 69, no. 1, 1 January 1980, pages 185-189, XP002051029**
- **N.TJANDRA ET AL.: "Solution NMR Measurement of Amide Proton Chemical Shift Anisotropy in 15-N-Enriched Proteins. Correlation with Hydrogen Bond Length" JOURNAL OF THE AMERICAN CHEMICAL SOCIETY, vol. 119, 27 August 1997, pages 8076-8082, XP002051030**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 916 963 B1

## Description

Attenuated $T_2$ relaxation by mutual cancellation of dipole-dipole coupling and chemical shift anisotropy indicates an avenue to NMR structures of very large biological macromolecules in solution.

**Abbreviations:** NMR, nuclear magnetic resonance; rf, radio-frequency; 2D, two-dimensional; FID, free induction decay; DD, dipole-dipole; CSA, chemical shift anisotropy; COSY, correlation spectroscopy; TROSY, transverse relaxation-optimized spectroscopy; PFG, pulsed field gradient; *ftz* homeodomain; *fushi tarazu* homeodomain polypeptide of 70 amino acid residues, with the homeodomain in positions 3-62.

Fast transverse relaxation of $^1H$, $^{15}N$ and $^{13}C$ by dipole-dipole coupling (DD) and chemical shift anisotropy (CSA) modulated by rotational molecular motions has a dominant impact on the size limit for biomacromolecular structures that can be studied by NMR spectroscopy in solution. TROSY (*T*ransverse *R*elaxation-*O*ptimized *S*pectroscop*Y*) is a new approach for suppression of transverse relaxation in multidimensional NMR experiments, which is based on constructive use of interference between DD coupling and CSA. For example, a TROSY-type two-dimensional $^1H$, $^{15}N$-correlation experiment with a uniformly $^{15}N$-labeled protein in a DNA complex of molecular weight 17 kDa at a $^1H$ frequency of 750 MHz showed that $^{15}N$ relaxation during $^{15}N$ chemical shift evolution and $^1H^N$ relaxation during signal acquisition are both significantly reduced by mutual compensation of the DD and CSA interactions. The reduction of the linewidths when compared with a conventional two-dimensional $^1H$, $^{15}N$-correlation experiment was 60% and 40%, respectively, and the residual linewidths were 5 Hz for $^{15}N$ and 15 Hz for $^1H^N$ at 4°C. Since the ratio of the DD and CSA relaxation rates is nearly independent of the molecular size, a similar percentagewise reduction of the overall transverse relaxation rates is expected for larger proteins. For a $^{15}N$-labeled protein of 150 kDa at 750 MHz and 20°C one predicts residual linewidths of 10 Hz for $^{15}N$ and 45 Hz for $^1H^N$, and for the corresponding uniformly $^{15}N$,$^2H$-labeled protein the residual linewidths are predicted to be smaller than 5 Hz and 15 Hz, respectively. The TROSY principle should benefit a variety of multidimensional solution NMR experiments, especially with future use of yet somewhat higher polarizing magnetic fields than are presently available, and thus largely eliminate one of the key factors that limit work with larger molecules.

## Introduction

Nuclear magnetic resonance (NMR) spectroscopy with proteins based on observation of a small number of spins with outstanding spectral properties, which may either be present naturally or introduced by techniques such as site-specific isotope labeling, yielded biologically relevant information on human hemoglobin (M = 65000) as early as 1969 (1), and subsequently also for significantly larger systems such as, for example, immunoglobulins (2). In contrast, the use of NMR for *de novo* structure determination (3, 4) has so far been limited to relatively small molecular sizes, with the largest NMR structure below molecular weight 30000. Although NMR in structural biology may, for practical reasons of coordinated use with X-ray crystallography (5), focus on smaller molecular sizes also in the future, considerable effort goes into attempts to extend the size limit to bigger molecules (for example, 6-8). Here we introduce "transverse relaxation-optimized spectroscopy" (TROSY) and present experimental data and theoretical considerations showing that this novel approach is capable of significantly reducing transverse relaxation rates and thus overcomes a key obstacle opposing solution NMR of larger molecules (7).

At the high magnetic fields typically used for studies of proteins and nucleic acids, chemical shift anisotropy interaction (CSA) of $^1H$, $^{15}N$ and $^{13}C$ nuclei forms a significant source of relaxation in proteins and nucleic acids, in addition to dipole-dipole (DD) relaxation. This leads to increase of the overall transverse relaxation rates with increasing polarizing magnetic field, $B_0$. Nonetheless, transverse relaxation of amide protons in larger proteins at high fields has been successfully reduced by complete or partial replacement of the non-labile hydrogen atoms with deuterons and, for example, more than 90 % of the $^{15}N$, $^{13}C^{\alpha}$ and $^1H^N$ chemical shifts were thus assigned in the polypeptide chains of a protein-DNA complex of size 64000 (6). TROSY uses spectroscopic means to further reduce $T_2$ relaxation based on the fact that cross-correlated relaxation caused by DD and CSA interference gives rise to different relaxation rates of the individual multiplet components in a system of two coupled spins 1/2, $I$ and $S$, such as, for example, the $^{15}N$-$^1H$ fragment of a peptide bond (9, 10). Theory shows that at $^1H$ frequencies near 1 GHz nearly complete cancellation of all transverse relaxation effects within a $^{15}N$-$^1H$ moiety can be achieved for one of the four multiplet components. TROSY observes exclusively this narrow component, for which the residual linewidth is then almost entirely due to DD interactions with remote hydrogen atoms in the protein. These can be efficiently suppressed by $^2H$-labeling, so that in TROSY-type experiments the accessible molecular size for solution NMR studies is no longer primarily limited by $T_2$ relaxation.

## Theory

We consider a system of two scalar coupled spins $\frac{1}{2}$, $I$ and $S$, with a scalar coupling constant $J_{IS}$, which is

located in a protein molecule. $T_2$ relaxation of this spin system is dominated by the DD coupling of $I$ and $S$ and by CSA of each individual spin, since the stereochemistry of the polypeptide chain restricts additional interactions of $I$ and $S$ to weak scalar and DD couplings with a small number of remote protons, $I_k$. The relaxation rates of the individual multiplet components of spin $S$ in a single quantum spectrum may then be widely different (9, 11, 12). They can be described using the single-transition basis operators $S^{\pm}_{12}$ and $S^{\pm}_{34}$ (13), which refer to the transitions 1→2 and 3→4 in the standard energy-level diagram for a system of two spins $\frac{1}{2}$, and are associated with the corresponding resonance frequencies, $\omega^{12}_S = \omega_S + \pi J_{IS}$ and $\omega^{34}_S = \omega_S - \pi J_{IS}$ (13-16):

$$\frac{d}{dt}\begin{bmatrix}\langle S^{\pm}_{12}\rangle \\ \langle S^{\pm}_{34}\rangle\end{bmatrix} = -\begin{bmatrix}\pm i\omega^{12}_S + R_{1212} + \frac{1}{T_{2S}} + \frac{1}{2T_{1I}} & 3(p^2 - \delta_I^2)J(\omega_I) - \frac{1}{2T_{1I}} \\ 3(p^2 - \delta_I^2)J(\omega_I) - \frac{1}{2T_{1I}} & \pm i\omega^{34}_S + R_{3434} + \frac{1}{T_{2S}} + \frac{1}{2T_{1I}}\end{bmatrix} \bullet \begin{bmatrix}\langle S^{\pm}_{12}\rangle \\ \langle S^{\pm}_{34}\rangle\end{bmatrix} \quad [1]$$

$\omega_S$ and $\omega_I$ are the Larmor frequencies of the spins $S$ and $I$, $T_{2S}$ and $T_{1I}$ account for the transverse relaxation of spin S and the longitudinal relaxation time of spin $I$, respectively, by all mechanisms of relaxation except DD coupling between the spins $S$ and $I$ and CSA of the spins $S$ and $I$.

$$p = \frac{1}{2\sqrt{2}} \gamma_I \gamma_S \hbar / r_{IS}^3,$$

$$\delta_S = \frac{1}{3\sqrt{2}} \gamma_S B_0 \Delta\sigma_S$$

and

$$\delta_I = \frac{1}{3\sqrt{2}} \gamma_I B_0 \Delta\sigma_I,$$

where $\gamma_I$ and $\gamma_S$ are the gyro-magnetic ratios of $I$ and $S$, $\hbar$ is the Planck constant divided by $2\pi$, $r_{IS}$ the distance between $S$ and $I$, $B_0$ the polarizing magnetic field, and $\Delta\sigma_S$ and $\Delta\sigma_I$ are the differences between the axial and the perpendicular principal components of the axially symmetric chemical shift tensors of spins $S$ and $I$, respectively. $R_{1212}$ and $R_{3434}$ are the transverse relaxation rates of the individual components of the $S$ doublet (11) given by Eqs. [2] and [3],

$$R_{1212} = (p - \delta_S)^2(4J(0) + 3J(\omega_S)) + p^2(J(\omega_I - \omega_S) + 3J(\omega_I) + 6J(\omega_I + \omega_S)) + 3\delta_I^2\, J(\omega_I) \quad [2]$$

$$R_{3434} = (p + \delta_S)^2(4J(0) + 3J(\omega_S)) + p^2(J(\omega_I - \omega_S) + 3J(\omega_I) + 6J(\omega_I + \omega_S)) + 3\delta_I^2\, J(\omega_I) \quad [3]$$

where $J(\omega)$ represents the spectral density functions at the frequencies indicated:

$$J(\omega) = \frac{2\tau_c}{5(1+(\tau_c\omega)^2)} \quad [4]$$

In deriving the Eqs. [2] and [3], parallel orientation of the principal symmetry axis of the chemical shift tensor and the vector $\boldsymbol{r}_{IS}$ was assumed. These equations show that whenever CSA and DD coupling are comparable, *i.e.*, $p \approx \delta_S$, the resonance at frequency $\omega^{12}_S$ may exhibit slow transverse relaxation even for very large molecules.

For a treatment of the relaxation of spin $I$ by Eq. [1] the symbols $I$ and $S$ can simply be interchanged. The single-transition operators $I^{\pm}_{13}$ and $I^{\pm}_{24}$ then refer to the transitions between the energy levels 1→3 and 2→4, respectively,

which are associated with the frequencies $\omega_I^{13} = \omega_I + \pi J_{IS}$ and $\omega_I^{24} = \omega_I - \pi J_{IS}$, and the relaxation rates $R_{1313}$ and $R_{2424}$ are determined by equations obtained by permutation of the *S* and *I* indices in Eqs. [2] and [3], respectively.

To evaluate the contributions from other mechanisms of relaxation we identify *I* and *S* as the $^1H^N$ and $^{15}N$ spins in a $^{15}N$-$^1H$ moiety. The relaxation of $^{15}N$ is then mainly determined by the CSA of $^{15}N$ and the DD interactions with the directly attached proton (17), so that the contributions from other interactions, $1/T_{1S}$ and $1/T_{2S}$, can to a good approximation be neglected. For $^1H^N$, $1/T_{1I}$ and $1/T_{2I}$ are dominated by DD interactions with other protons $I_k$ at distance $r_k$. These can be accounted for by spectral density functions $J_k(\omega)$, which describe the motions of the vectors joining the individual $^1H^N$-$^1H_k$ spin pairs (17):

$$1/T_{1I} = \sum_k (\gamma_I^2 \hbar / 2r_k^3)^2 (J_k(0) + 3J_k(\omega_I) + 6J_k(2\omega_I)), \quad [5]$$

$$1/T_{2I} = \sum_k (\gamma_I^2 \hbar / 2r_k^3)^2 (\frac{5}{2} J_k(0) + \frac{9}{2} J_k(\omega_I) + 3J_k(2\omega_I)). \quad [6]$$

Here, the equations [1] to [6] were used to calculate theoretical lineshapes of spin multiplets for given sets of the relaxation parameters, which were subsequently compared with the experimental NMR data. In particular, the in-phase absorptive spectrum was calculated using Eq. [7] (14),

$$I(\omega) = \mathrm{Re}[\mathbf{V}\,\mathbf{A}^{-1}\mathbf{V}], \quad [7]$$

where **V** = (1,1) and the relaxation matrix **A** is the (2x2) matrix on the right hand side of Eq. [1].

## Experimental procedures

NMR spectra were recorded on Bruker DRX 750 and Varian Unity*plus* 400 spectrometers with a 2 mM solution of the specific 1:1 complex formed between a uniformly $^{15}N$-labeled 70-residue *fushi tarazu (ftz)* homeodomain polypeptide and an unlabeled 14-base pair DNA duplex (18, 19) in 95% $^1H_2O$/5% $^2H_2O$ at pH 6.0 and 4°C.

The isotropic rotational correlation time, $\tau_c$, of the complex was estimated from the $T_1/T_2$ ratio of the relaxation times of the backbone $^{15}N$ nuclei (20). The experimental schemes of Farrow *et al.* (21) were used for measurements of $T_1(^{15}N)$ and $T_2(^{15}N)$ for backbone nitrogen atoms.

The TROSY (*T*ransverse *R*elaxation-*O*ptimized *S*pectroscop*Y*) approach (Fig. 1) and conventional [$^{15}N$,$^1H$]-COSY (22, 23) experiments were used to correlate $^1H$ and $^{15}N$ resonances. For all spectra $t_{1\,max}$ = 90 ms and $t_{2\,max}$ = 171 ms were used. In TROSY the evolution of the *I*,*S* spin system due to the $^1J_{IS}$ scalar coupling was not refocused during $t_1$ and $t_2$, thus avoiding suppression of cross-correlated relaxation during these periods. To obtain the pure absorptive spectrum containing only the most slowly relaxing component of the 2D multiplets, the scheme of Fig. 1 was employed (see also the Appendix: Quantitative analysis of TROSY).

## Results

The NMR experiments with the uniformly $^{15}N$-labeled *ftz* homeodomain complexed with a 14-base pair DNA duplex were performed at 4°C. The $T_1/T_2$ ratio of $^{15}N$ was used to estimate the effective global correlation time, $\tau_c$, of the complex (20). For the backbone amide groups, average $T_1(^{15}N)$ and $T_2(^{15}N)$ values of 0.720 ± 0.03 and 0.042 ± 0.005 s, respectively, were measured at 400 MHz, resulting in a global rotational correlation time of $\tau_c$ = 20 ± 2 ns. This $\tau_c$ value corresponds to that expected for a spherical protein of size 40 kDa in $H_2O$ solution at 35°C.

The figure 2 shows a small region from $^{15}N$-$^1H$ correlation spectra of the *ftz* homeodomain-DNA complex that contains the resonance of the indole $^{15}N$-$^1H$ moiety of Trp48, which is buried in the core of the protein (19). In the conventional [$^{15}N$,$^1H$]-COSY experiment (22, 23), decoupling of $^1H$ and $^{15}N$ during the time periods $t_1$ and $t_2$, respectively, leads to detection of a single correlation peak per $^{15}N$-$^1H$ moiety (Fig. 2a). If the same [$^{15}N$, $^1H$]-COSY spectrum is recorded without decoupling, four cross peaks are observed per $^{15}N$-$^1H$ moiety, which show largely different linewidths (Fig. 2b). The cross peak at ($\omega_1$ = 130.7 ppm, $\omega_2$ = 10.78 ppm) exhibits the broadest linewidths in both dimensions, which shows that it originates from the rapidly relaxing components of both $^1H^N$ and $^{15}N$. One-dimensional cross sections taken along $\omega_2$ and $\omega_1$ at the positions indicated by arrows in the spectra presented in Fig. show that the two

cross peaks at ($\omega_1$ = 132.1 ppm, $\omega_2$ = 10.78 ppm) and ($\omega_1$ = 130.7 ppm, $\omega_2$ = 10.65 ppm) are broadened either along $\omega_1$ or along $\omega_2$ (Fig. 3). The cross peak at ($\omega_1$ = 132.1 ppm, $\omega_2$ = 10.65 ppm) displays narrow linewidths in both dimensions, showing that it originates from the two slowly relaxing components of the $^{15}N$-$^1H$ doublets. The TROSY-type correlation experiment, which does not use decoupling either during $t_1$ or $t_2$, contains only this narrowest correlation peak (Fig. 2c), which shows about 60% and 40% decrease in the linewidths of the $^{15}N$ and $^1H$ resonances, respectively, when compared to the collapsed cross peak in the conventional, broadband-decoupled spectrum (Fig. 2).

The fits of the experimental line shapes shown in Fig. 3 were obtained with line shape calculations using the parameters $\tau_c$ = 20 ns and $^1J(^1H,^{15}N)$ = 105 Hz, where the chemical shift anisotropies, $\Delta\sigma_H$ and $\Delta\sigma_N$, were adjusted for the best fit. Since there was an otherwise unaccountable deviation from the Lorentzian lineshape we included a long-range scalar coupling $^2J(^1H^{\delta1},^{15}N^{\varepsilon1})$=-5 Hz (24) in the calculations, and $T_1$ and $T_2$ relaxation of $^1H^N$ due to dipole-dipole coupling with other protons was modeled by placing 3 protons at a distance of 0.24 nm from $^1H^N$. Application of one or a series of 180° pulses on spin *I* during the evolution of spin *S* interchanges the slowly and rapidly relaxing components of the *S* multiplet, which results in averaging of the slow and fast relaxation rates and elimination of the CSA/DD interference (25, 26). Indeed, the line shapes of the $^1H^N$ and $^{15}N$ resonances measured with conventional [$^{15}N$, $^1H$]-COSY are well reproduced if the average of the two relaxation rates is used in the simulation (Fig. 3, a1 and b1). The best fit values of $\Delta\sigma_H$=-16 ppm and $\Delta\sigma_N$= - 160 ppm correspond closely to the experimentally measured chemical shift anisotropies of $^1H$ and $^{15}N$ in $^{15}N$-$^1H$ moieties: with solid state NMR studies of $^{15}N$-$^2D$ moieties, values for $\Delta\sigma_D$ near -14 ppm (27) and for $\Delta\sigma_N$ of -160 ppm (28) were determined; independently, solution NMR experiments yielded values for $\Delta\sigma_H$ of backbone amide protons in the range -8 to -17 ppm (A. Bax, personal communication) and $\Delta\sigma_N$ near -170 ppm (10).

**Discussion**

In the experiments with the *ftz* homeodomain-DNA complex described in this paper the overall transverse relaxation rates of $^{15}N$ and $^1H^N$ in the indole $^{15}N$-$^1H$ moiety of a buried tryptophan were reduced by 60% and 40%, respectively, when using a TROSY-type [$^{15}N$,$^1H$]-correlation experiment instead of the conventional [$^{15}N$,$^1H$]-COSY scheme. At a first glance this may appear to be a modest improvement, but a closer look reveals that DD coupling with remote protons, which could be nearly completely suppressed by replacement of the non-labile hydrogen atoms with $^2H$ (*e.g.*, 6,8), accounts for 95% of the residual $T_2(^1H^N)$ relaxation and 75% of the residual $T_2(^{15}N)$ relaxation. In a corresponding DNA complex with the perdeuterated *ftz* homeodomain the reduction of the $T_2$ relaxation rates of the $^{15}N$-$^1H$ moieties by the use of TROSY at 750 MHz would be about 40-fold for $^1H^N$ and about 10-fold for $^{15}N$.

Using the equations [1]-[6] with $\Delta\sigma_H$= -16 ppm, $\Delta\sigma_N$=-160 ppm, $r_{HN}$= 0.101 nm and parallel orientation of the principal axis of the CSA tensor with the vector $\boldsymbol{r}_{HN}$ we evaluated the dependence of the residual $T_2$ relaxation rates of $^{15}N$ and $^1H$ in TROSY-type experiments on the polarizing magnetic field $B_0$ and the molecular size. These calculations showed that nearly complete compensation of $T_2$ relaxation due to DD and CSA within the $^{15}N$-$^1H$ moieties is obtained at a $B_0$ strength corresponding to a $^1H$ frequency near 1100 MHz, *i.e.*, at this field strength ($p$ - $\delta_S$) $\cong$ 0 and ($p$ -$\delta_I$) $\cong$ 0 in Eq. [2]. Theory further predicts that the residual TROSY $T_2$ relaxation rates due to DD and CSA interactions within the $^{15}N$-$^1H$ fragment are practically independent of the molecular size. For perdeuterated proteins the size limit for TROSY-type [$^{15}N$,$^1H$]-correlation experiments is thus not critically determined by $T_2$ relaxation, but one needs nonetheless to consider that the effect of deuteration of the nonlabile proton sites in the protein is dependent on conformation. For the $^{15}N$-$^1H$ moieties in $\beta$-sheet secondary structure, DD and CSA interactions within the $^{15}N$-$^1H^N$ fragment are the only sources of transverse relaxation that need to be considered, whereas in $\alpha$-helices the two sequentially adjacent $^1H^N$ protons (3) contribute significantly to the transverse relaxation of the $^{15}N$ and $^1H^N$ spins.

In order to provide a tangible illustration (Fig. 4) we calculated the $^1H^N$ and $^{15}N$ line shapes for two perdeuterated spherical proteins in $^1H_2O$ solution with rotational correlation times $\tau_c$ of 60 and 320 ns, which corresponds to molecular weights of 150 kDa and 800 kDa, respectively. A magnetic field $B_0$ corresponding to a resonance frequency 750 MHz for protons was assumed. To account for the worst possible situation for DD interaction with remote protons, two protons were placed at 0.29 nm from $^1H^N$. The linewidth of the narrow component of the $^{15}N$ doublet increases only slightly with molecular weight and is about 5 Hz at 150 kDa and 15 Hz for a 800 kDa protein (Fig. 4). The $^1H^N$ linewidth depends more strongly on the residual DD interactions with remote protons and is about 15 Hz at 150 kDa and 50 Hz for a 800 kDa protein. For the 150 kDa protein these numbers correspond to 10-fold and 4-fold reduction of the $^{15}N$ and $^1H^N$ TROSY linewidths, respectively, when compared with a conventional [$^{15}N$,$^1H$]-COSY experiment with broad-band decoupling of $^{15}N$ and $^1H$. For large molecular sizes the experimental scheme of Fig. 1 may in principle be further improved by elimination of the 180° refocusing rf-pulses during the INEPT transfers, since during the INEPT mixing times these pulses mix the multiplet components with slow and fast $T_2$ relaxation in a similar way as during the entire experiment in conventional [$^{15}N$,$^1H$]-COSY. The elimination of decoupling sequences and 180° pulses from TROSY-type NMR pulse sequences may also have implications for future probe designs, since the constraints by the requirements for minimal radio frequency heating and maximal $B_1$ homogeneity may then be relaxed, permitting a

better optimization of other paramters such as sensitivity or sample diameter.

The TROSY principle drastically reduces all major sources of relaxation thoughout the entire NMR experiment, including signal acquisition, and is clearly distinct from the use of heteronuclear multiple-quantum coherence to reduce dipolar relaxation between heteronuclei (29), which was previously used for measurements of $^3J_{H\alpha H\beta}$ scalar coupling constants in proteins (30). Heteronuclear multiple-quantum coherences are subject to dipolar relaxation with remote spins as well as to CSA relaxation, which limits the use of these coherences at high polarizing magnetic fields. Moreover, the slow relaxation of the multiple-quantum coherences cannot be used during signal acquisition (14), which is critical for large molecules.

The following are some initial considerations on practical applications of the TROSY principle. (*i*) Since only one of the four multiplet components of $^{15}$N-$^1$H moiety is retained in TROSY-type experiments, the conventional [$^{15}$N,$^1$H]-COSY is intrinsically more sensitive. However, for measurements with proteins at $^1$H frequencies higher than 500 MHz, TROSY will provide a much better ratio of signal height to noise. (*ii*) TROSY-type [$^{13}$C,$^1$H]-correlation experiments with the $^{13}$C-$^1$H moieties of the aromatic rings of Tyr, Phe and Trp yield comparable results to those for $^{15}$N-$^1$H moieties (unpublished results). (*iii*) 2D NOESY experiments correlating amide protons and aromatic protons can be relayed by TROSY-type heteronuclear correlation experiments. In favorable cases this might result in low resolution structures for several-fold larger proteins than have been accessible so far. (*iv*) We anticipate that a wide variety of NMR experiments currently employed for resonance assignments and collection of conformational constraints can be optimized for larger molecular sizes by use of the TROSY approach in one or several dimensions.

## Appendix: Quantitative analysis of TROSY

The coherence transfer during the pulse sequence of Fig. 1 was evaluated using the product operator formalism (31) as implemented in the program POMA (32), and the resulting phases of the rf-pulses and the receiver were transferred into the experimental pulse program according to (33). The transverse proton magnetization after the first 90° pulse on protons (***a*** in Fig. 1) is then given by Eq. [8]:

$$\sigma(a) = -I_y. \qquad [8]$$

During the delay $2\tau_1$ the scalar coupling $^1J(^1H,^{15}N)$ evolves, so that the first 90°($^{15}$N) pulse generates two-spin coherence. With $\tau_1 = 1/(4\,^1J(^1H,\,^{15}N))$ we have at time ***b*** for the first step of the phase cycle (Fig. 1):

$$\sigma_1(b) = 2I_zS_x = I_zS^- + I_zS^+ \qquad (\psi_1 = y) \qquad [9]$$

The evolution of these terms during $t_1$, including relaxation, was evaluated using the single-transition basis operators $S_{12}^{\pm}$ and $S_{34}^{\pm}$ :

$$S_{12}^{\pm} = \frac{1}{2}S^{\pm} + I_zS^{\pm} \qquad [10]$$

$$S_{34}^{\pm} = \frac{1}{2}S^{\pm} - I_zS^{\pm} \qquad [11]$$

The time evolution of the expectation values of these operators can be obtained by integration of Eq. [1] with initial conditions derived from Eq. [9] and the assumption that $1/T_{1I} \ll {}^1J(^1H,\,^{15}N)$, which results in the following density matrix at time ***c***:

$$\begin{aligned}\sigma_1(c) = {} & \frac{1}{2}S^-(exp[i\omega_S^{12}\,t_1 - R_{12}\,t_1] - exp[i\omega_S^{34}\,t_1 - R_{34}\,t_1]) + \\ & I_zS^-(exp[i\omega_S^{12}\,t_1 - R_{12}t_1] + exp[i\,\omega_S^{34}\,t_1 - R_{34}t_1]) + \\ & \frac{1}{2}S^+(exp[-i\omega_S^{12}\,t_1 - R_{12}\,t_1] - exp[-i\omega_S^{34}\,t_1 - R_{34}t_1]) \\ & + I_zS^+(exp[-i\omega_S^{12}\,t_1 - R_{12}\,t_1] + exp[-i\omega_S^{34}\,t_1 - R_{34}\,t_1])\end{aligned} \qquad [12]$$

The relaxation factors $R_{ij}$ are related to the individual relaxation rates of the multiplet components by Eqs. [13] and [14]:

$$R_{jk} = R_{jkjk} + 1/T_{2S} + 1/(2T_{1I}) \qquad (jk = 12,34) \qquad [13]$$

$$R_{jk} = R_{jkjk} + 1/T_{2I} + 1/(2T_{1S}) \qquad (jk = 13,24) \qquad [14]$$

The subsequent polarization transfer step (time period ***c*** to ***d*** in Fig. 1) links the evolution period $t_1$ with the acquisition period $t_2$. The density matrix at time point ***c*** is represented by Eq. [15], where only those $\Gamma$ and $\Gamma S_z$ coherences are retained that result in detectable signals during data acquisition:

$$\begin{aligned} \sigma_1(d) = {} & \frac{1}{2}\Gamma(i\,cos[\omega_S^{12}\,t_1] - sin[\omega_S^{12}\,t_1])exp[-R_{12}t_1] + \\ & \frac{1}{2}\Gamma(i\,cos[\omega_S^{34}\,t_1] + sin[\omega_S^{34}\,t_1])exp[-R_{34}t_1] - \\ & \Gamma S_z(i\,cos[\omega_S^{12}\,t_1] - sin[\omega_S^{12}\,t_1])exp[-R_{12}t_1] + \\ & \Gamma S_z(i\,cos[\omega_S^{34}\,t_1] + sin[\omega_S^{34}\,t_1])exp[-R_{34}t_1] \end{aligned} \qquad [15]$$

The other steps in the phase cycle of Fig. 1 can be analysed in an analogous fashion. Accumulation of the 8 transients of the pulse sequence results in the following spectral density matrix for the real part of the interferogram:

$$\sigma_{Re}(d) = -4\Gamma sin[\omega_S^{12}\,t_1]\,exp[-R_{12}t_1] + 8\Gamma S_z sin[\omega_S^{12}\,t_1]exp[-R_{12}t_1] \qquad [16]$$

Incrementation of the phase $\psi_1$ by 90° at each discrete value of $t_1$ leads to the corresponding imaginary part:

$$\sigma_{Im}(d) = -4\Gamma cos[\omega_S^{12}\,t_1]\,exp[-R_{12}t_1] + 8\Gamma S_z cos[\omega_S^{12}\,t_1]exp[-R_{12}t_1] \qquad [17]$$

The equations [16] and [17] are combined to the hypercomplex interferogram that represents pure phase correlation in the $\omega_1$ dimension.

The treatment of the relaxation of the $^1H^N$ coherences during the acquisition period $t_2$ is similar to the treatment of $^{15}N$ during $t_1$ (see Theory). The signals generated by $\Gamma$ and $\Gamma S_z$ coherences that are received during $t_2$ are described by Eqs. [18] and [19], respectively:

$$\Gamma \rightarrow A\,(exp[i\omega_I^{13}\,t_2 - R_{13}t_2] + exp[i\omega_I^{24}\,t_2 - R_{24}t_2]) \qquad [18]$$

$$2\Gamma S_z \rightarrow A\,(exp[i\omega_I^{13}\,t_2 - R_{13}t_2] - exp[i\omega_I^{24}\,t_2 - R_{24}t_2]), \qquad [19]$$

where $A$ is a proportionality coefficient. Substitution of Eqs. [18] and [19] into Eqs. [16] and [17] results in the hypercomplex interferogram corresponding to the $1\rightarrow2$ and $2\rightarrow4$ transitions of the $^1H$, $^{15}N$ spin system:

$$\sigma_{1224} = 8\,A\,exp[i\omega_S^{12}\,t_1 + i\omega_I^{24}\,t_2]exp[-(R_{12}t_1 + R_{24}t_2)] \qquad [20]$$

Finally, the Fourier transformation of the hypercomplex interferogram represented by Eq. [20] results in the pure absorptive correlation spectrum, with resonance frequencies in $\omega_1$ and $\omega_2$ corresponding to the desired individual component of the $^{15}N$-$^1H$ multiplet.

**References**

1. Shulman, R.G., Ogawa, S., Wüthrich, K., Yamane, T., Peisach, J. & Blumberg, W.E. (1969) *Science* **165**, 251-257.
2. Arata, Y., Kato, K., Takahashi, H. & Shimada, I. (1994) *Methods in Enzymology* **239**, 440-464.
3. Wüthrich, K. (1986) *NMR of Proteins and Nucleic Acids* (Wiley, New York).
4. Wüthrich, K. (1995) *NMR in Structural Biology* (World Scientific, Singapore).
5. Wüthrich, K. (1995) *Acta Cryst.* **D 51**, 249-270.
6. Shan, X., Gardner, K. H., Muhandiram, D. R., Rao, N. S., Arrowsmith, C. H. & Kay, L. E. (1996) *J. Am. Chem. Soc.* **118**, 6570-6579.
7. Wagner, G. (1993) *J. Biomol. NMR* **3**, 375-385.
8. Nietlispach, D., Clowes, R.T., Broadhurst, R.W., Ito, Y., Keeler, J., Kelly, M., Ashurst, J., Oschkinat, H., Domaille, P.J. & Laue, E.D. (1996) *J. Am. Chem. Soc.* **118**, 407-415.
9. Guéron, M., Leroy, J.L. & Griffey, R.H. (1983) *J. Am. Chem. Soc.* **105**, 7262-7266.
10. Tjandra, N., Szabo, A. & Bax, A. (1996) *J. Am. Chem. Soc.* **118**, 6986-6991.
11. Farrar, T.C. & Stringfellow, T.C. (1996) in *Encyclopedia of NMR*, eds. Grant, D.M. & Harris, R.K. (Wiley, New York), Vol. **6**, pp. 4101-4107.
12. Void, R.R. & Vold, R.L. (1978) *Prog. NMR Spectrosc.* **12**, 79-133.
13. Ernst, R.R., Bodenhausen, G. & Wokaun, A. (1987) *The Principles of Nuclear Mangetic Resonance in One and Two Dimensions* (Clarendon Press, Oxford).
14. Abragam, A. (1961) *The Principles of Nuclear Magnetism.* (Clarendon Press, Oxford).
15. Goldman, M. (1984) *J. Magn. Reson.* **60**, 437-452.
16. London, R.E. (1990) *J. Magn. Reson.* **86**, 410-415.
17. Peng, J.W. & Wagner, G. (1992) *J. Magn. Reson.* **98**, 308-332.
18. Percival-Smith, A., Müller, M., Affolter, M. & Gehring, W.J. (1990) *EMBO J.* **9**, 3967-3974.
19. Qian, Y.Q., Furukubo-Tokunaga, K., Resendez-Perez, D., Müller, M., Gehring, W.J. & Wüthrich K. (1994) *J. Mol. Biol.* **238**, 333-345.
20. Kay, L. E., Torchia, D. A. & Bax, A. (1989) *Biochemistry* **28**, 8972-8979.
21. Farrow, N. A., Muhandiram, R., Singer, A. U., Pascal, S. M., Kay, C.M., Gish, G., Shoelson, S. E., Pawson, T., Forman-Kay, J. D., & Kay, L. E. (1994) *Biochemistry* **33**, 5984-6003.
22. Müller, L, (1979) *J. Am. Chem. Soc.* **101**, 4481-4484.
23. Bodenhausen, G. & Ruben, D.J. (1980) *Chem. Phys. Lett.* **69**, 185-189.
24. Bystrov, V.F. (1976) *Prog. NMR Spectrosc.* **10**, 41-81.
25. Palmer, A. G., Skelton, N. J., Chazin, W. J., Wright, P. E. & Rance, M. (1992) *Mol. Phys.* **75**, 699-711.
26. Kay, L. E., Nicholson, L.K., Delaglio, F., Bax, A. & Torchia, D. A. (1992) *J. Magn. Reson.* **97**, 359-375.
27. Michal, C.A., Wehman, J.C., Jelinski, L.W., (1996) *J. Magn. Reson. Ser. B.* **111**, 31-39.
28. Hiyama, Y., Niu, C., Silverton, J. V., Bavoso, A. & Torchia, D. A. (1988) *J. Am. Chem. Soc.* **110**, 2378-2383.
29. Griffey, R.H. & Redfield, A.G. (1987) *Quart. Rev. Biophys.* **19**, 51-82.
30. Grzesiek, S., Kuboniwa, H., Hinck, A.P. & Bax A. (1995) *J. Am. Chem. Soc.* **117**, 5312-5315.
31. SØrensen, O.W., Eich, G.W., Levitt, M.H., Bodenhausen, G. & Ernst R.R. (1983) *Prog. NMR Spectrosc.* **16**, 163-192.
32. Güntert, P., Schaefer, N., Otting, G. & Wüthrich, K. (1993) *J. Magn. Reson.* **101**, 103-105.
33. Levitt, M.H. (1997) *J. Magn. Reson.* **126**, 164-182.
34. Piotto, M., Saudek, V. & Sklenar, V.J. (1992) *J. Biomol. NMR* **2**, 661-665.


**Figure captions**

**Figure 1.** Experimental scheme for TROSY-type 2D $^{1}H$,$^{15}N$ correlation spectroscopy. In the rows marked $^{1}N$ and $^{15}N$, narrow and wide bars stand for non-selective 90° and 180° rf pulses, respectively. Water suppression is achieved by WATERGATE (34), using the two off-resonance rf pulses indicated by curved shapes. The $^{1}H$ and $^{15}N$ carrier frequencies are placed at 9 and 127 ppm, respectively. The delay $\tau_1$ corresponds to $1/(4^{1}J(^{1}H,^{15}N))$ = 2.7 ms. Phases used are $\psi_1$={y, -y, -x, x, y, -y, -x, x} ; $\psi_2$={4(x), 4(-x)} ; $\phi_1$={4(y), 4(-y)} ; $\phi_2$(receiver)= {x, -x, -y, y, x, -x, y, -y} ; x on all other pulses. The row marked PFG indicates the applied magnetic field gradients along the z-axis: $G_1$, amplitude = 30 G/cm, duration = 0.4 ms; $G_2$, - 60 G/cm, 1 ms; $G_3$, 50 G/cm, 0.4 ms; $G_4$, 48 G/cm, 0.6 ms. Two FIDs are recorded per $t_1$ delay, with $\psi_1$ incremented by 90° in-between, and stored as the real and imaginary parts of the interferogram in $t_1$. The Fourier transformation results in a 2D $^{1}H$, $^{15}N$ correlation spectrum which contains only the component of

the four-line $^{15}N$-$^{1}H$ multiplet that has the slowest $T_2$ relaxation rates for both nuclei.

**Figure 2.** Contour plots of $^{15}N$, $^{1}H$ correlation spectra showing the indole $^{15}N$-$^{1}H$ spin system of Trp 48 recorded in a 2 mM solution of uniformly $^{15}N$-labeled *ftz* homeodomain complexed with an unlabeled 14-bp DNA duplex in 95% $H_2O$/5% $^2H_2O$ at 4°C, pH = 6.0, measured at the $^{1}H$ frequency of 750 MHz. (a) Conventional broad-band decoupled [$^{15}N$, $^{1}H$]-COSY spectrum (22, 23). The evolution due to the $^{1}J(^{1}H,^{15}N)$ scalar coupling was refocused in the $\omega_1$ and $\omega_2$ dimensions by a 180° proton pulse in the middle of the $^{15}N$ evolution time $t_1$, and by WALTZ composite pulse decoupling of $^{15}N$ during data acquisition, respectively; (b) conventional [$^{15}N$,$^{1}H$]-COSY spectrum recorded without decoupling during $t_1$ and $t_2$; (c) TROSY-type $^{15}N$,$^{1}H$ correlation spectrum recorded with the pulse scheme of Fig. 1. Chemical shifts relative to DSS in ppm and shifts in Hz relative to the center of the multiplet are indicated in both dimensions. The arrows identify the locations of the cross sections shown in Fig. 3.

**Figure 3.** Cross sections through the spectra of Fig. 2 (solid lines). To facilitate a comparison of the linewidths in the different spectra the cross sections were normalized to the same maximal signal amplitude. (a1), (a2), ... refer to the arrows in Fig. 2. Simulated line shapes (dashed lines in (a) and (b)) were calculated using $1J(^{1}H,^{15}N) = -105$ Hz, a rotational correlation time of $\tau_c = 20$ ns, and chemical shift anisotropies of $\Delta\sigma_H = -16$ ppm and $\Delta\sigma_N$=-160 ppm. A long-range scalar coupling $^{2}J(^{1}H^{\delta1},^{15}N^{\varepsilon1})$ =- 5 Hz was included in the simulation of the $^{15}N$ lineshapes (24), but possible effects of the small scalar couplings $^{3}J(^{1}H^{\delta1},^{1}H^{\varepsilon1})$ and $^{3}J(^{1}H^{\zeta2},^{15}N^{\varepsilon1})$ were neglected. For $^{1}H^{N}$ the relaxation due to DD coupling with other protons in the non-deuterated complex was approximated by 3 protons placed at a distance of 0.24 nm from $^{1}H^{N}$.

**Figure 4.** $^{1}H$ and $^{15}N$ lineshapes predicted for the broad and narrow multiplet components of $^{1}H^{N}$ and $^{15}N$ of the $^{15}N$-$^{1}H$ moiety in a [$^{15}N$,$^{1}H$]-COSY experiment of the type of Fig. 3 b1 and b2 for large proteins in $H_2O$ solution at 20°C and a $^{1}H$ frequency of 750 MHz. (a1) and (a2): Spherical protein of size 150 kDa. For the calculation a rotational correlation time of 60 ns, $\Delta\sigma_H$=- -16 ppm and $\Delta\sigma_N$ = -160 ppm were used, and all nonlabile protons were replaced with deuterons. Relaxation due to DD coupling with other labile protons was modelled by placing two protons at a distance of 0.29 nm from $^{1}H^{N}$. The full linewidths at half height are indicated. (b1) and (b2): Spherical protein of size 800 kDa. The calculation used $\tau_c$ = 320 ns and otherwise the same parameters as in (a).

**Claims**

1. A method for obtaining a nuclear magnetic resonance (NMR) correlation spectrum of heteronuclear spin systems, in particular comprising large molecules, especially biological macromolecules in solution, the spin system being subjected to a homogeneous magnetic field $B_0$, being irradiated by a sequence of radio frequency (rf) pulses and comprising at least two kinds of spin 1/2 nuclei I and S being coupled to each other,
characterized in that
the sequence of rf pulses is chosen such that line broadening in the observed spectrum due to transverse relaxation ($T_2$) is significantly reduced because of cross correlation between dipole-dipole (DD) coupling of the spins and chemical shift anisotropy (CSA), giving rise to different relaxation rates of the individual multiplet components of the spin system, whereby exclusively the NMR signals of the multiplet component are recorded, for which the contributions to the transverse relaxation rate from DD and CSA interactions have different signs.

2. The method of claim 1, characterized in that the sequence of rf pulses comprises a first non-selective 90° pulse affecting the first kind of spin 1/2 nuclei I, followed by a delay time $\tau_1$, two non-selective 180° pulses affecting spin 1/2 nuclei I and S, respectively, followed by another delay time $\tau_1$, a second non-selective 90° pulse, affecting the first kind of nuclei I, a first phase-cycled 90° pulse $\psi_1$ acting on the second kind of nuclei S, followed by an evolution time $t_1$, a first phase-cycled 90° pulse $\varphi_1$ acting on the first kind of nuclei I, followed by a delay time $\tau_1$, two non-selective 180° pulses affecting both kinds of nuclei I and S, respectively, followed by another delay time $\tau_1$, two non-selective 90° pulses affecting nuclei I and S, respectively, followed by a delay time $\tau_1$, two non-selective 180° pulses affecting nuclei I and S, respectively, followed by a delay time $\tau_1$ and a second phase-cycled pulse $\psi_2$ acting on the second kind of nuclei S, followed by data acquisition during a time period $t_2$.

3. The method of claim 2, characterized in that magnetic field gradients $G_1$, $G_3$, $G_4$ of different strength are applied at least during parts of the delay time intervals $\tau_1$ and that a magnetic field gradient $G_2$ is applied just before irradiating the first phase-cycled pulse $\psi_1$ acting on the second kind of nuclei, S.

4. The method of claims 2 or 3, characterized in that no refocussing actions are performed with respect to the I, S spin systems during the evolution time interval $t_1$ and the acquisition time period $t_2$, thus avoiding averaging of the different relaxation rates and elimination of the DD and CSA interference during these periods

5. The method of claim 4, characterized in that the 180° refocussing rf pulses during INEPT transfer and all evolution periods are eliminated in a suitable rf pulse sequence.

6. The method of one of the previous claims, characterized in that the sequence of rf pulses comprises a part adapted to suppress NMR signals of a solvent.

7. The method of claim 6, characterized in that the sequence of rf pulses comprises a part adapted to suppress NMR signals of water, especially a so-called "WATERGATE" subsequence comprising two off-resonance rf pulses at the end of the sequence just before data acquisition.

8. The method of one of the previous claims, characterized in that the residual linewidth is almost entirely due to DD interactions with remote hydrogen atoms in a protein and that the DD interactions are reduced by $^2$H-labelling.

9. The method of one of the previous claims, characterized in that the obtained heteronuclear correlation spectrum is used for relaying by a second experiment, in particular by a NOESY experiment correlating amide protons and aromatic protons.

**Patentansprüche**

1. Verfahren zum Erhalten eines Kernspinresonanz-(NMR)-Korrelationsspektrums von heteronuklearen Spinsystemen, die insbesondere große Moleküle umfassen, speziell biologische Makromoleküle in Lösung, wobei das Spinsystem einem homogenen Magnetfeld $B_0$ ausgesetzt wird, welches mit einer Sequenz von Hochfrequenz-(HF)-Impulsen bestrahlt wird und mindestens zwei Sorten von Spin-1/2-Kernen I und S aufweist, die miteinander gekoppelt sind,
dadurch gekennzeichnet,
daß die Sequenz der HF-Impulse so gewählt ist, daß die Linienverbreiterung in dem beobachteten Spektrum aufgrund transversaler Relaxation ($T_2$) signifikant reduziert wird aufgrund von Kreuz-Korrelation zwischen Dipol-Dipol-(DD)-Kopplung der Spins und Chemische-Verschiebung-Anisotropie (CSA), wodurch unterschiedliche Relaxationsraten der einzelnen Multiplet-Komponenten des Spinsystems entstehen, wodurch ausschließlich die NMR-Signale derjenigen Multiplet-Komponente aufgezeichnet werden, für welche die Beiträge zur transversalen Relaxationsrate von DD- und CSA-Wechselwirkungen unterschiedliche Vorzeichen haben.

2. Verfahren nach Anspruch 1, dadurch gekennzeichnet, daß die Sequenz von HF-Impulsen aufweist: einen ersten nicht-selektiven 90°-Impuls, welcher die erste Sorte von Spin-1/2-Kernen I beeinflußt, gefolgt von einer Verzögerungszeit $\tau_1$, zwei nicht-selektive 180°-Impulse, die die Spin-1/2-Kerne I bzw. S beeinflussen, gefolgt von einer weiteren Verzögerungszeit $\tau_1$, einen zweiten nicht-selektiven 90°-Impuls, welcher die erste Kernsorte I beeinflußt, einen ersten phasenzyklierten 90°-Impuls $\psi_1$, welcher auf die zweite Kernsorte S wirkt, gefolgt von einer Evolutionszeit $t_1$, einen ersten phasenzyklierten 90°-Impuls $\varphi_1$, welcher auf die erste Kernsorte I wirkt, gefolgt von einer Verzögerungszeit $\tau_1$, zwei nicht-selektive 180°-Impulse, welche beide Kernsorten I bzw. S beeinflussen, gefolgt von einer weiteren Verzögerungszeit $\tau_1$, zwei nicht-selektive 90°-Impulse, welche die Kerne I bzw. S beeinflussen, gefolgt von einer Verzögerungszeit $\tau_1$, zwei nicht-selektive 180°-Impulse, welche die Kerne I bzw. S beeinflussen, gefolgt von einer Verzögerungszeit $\tau_1$ und einen zweiten phasenzyklierten Impuls $\psi_2$, welcher auf die zweite Kernsorte S wirkt, gefolgt von einer Datenerfassung während einer Zeitperiode $t_2$.

3. Verfahren nach Anspruch 2, dadurch gekennzeichnet, daß Magnetfeldgradienten $G_1$, $G_3$, $G_4$ von unterschiedlichen Stärken mindestens während Teilen der Verzögerungszeitintervalle $\tau_1$ angelegt werden, und daß ein Magnetfeldgradient $G_2$ kurz vor dem Einstrahlen des ersten phasenzyklierten Impulses $\psi_1$, welcher auf die zweite Kernsorte S wirkt, angelegt wird.

4. Verfahren nach Anspruch 2 oder 3, dadurch gekennzeichnet, daß keine Refokussieraktionen in bezug auf die I-, S-Spinsysteme während des Evolutions-Zeitintervalls $t_1$ und der Erfassungs-Zeitperiode $t_2$ ausgeführt werden, wodurch eine Mittelung der unterschiedlichen Relaxationsraten und Elimination der DD- und CSA-Interferenz während dieser Perioden vermieden wird.

5. Verfahren nach Anspruch 4, dadurch gekennzeichnet, daß die 180°-Refokussierungs-HF-Impulse während eines INEPT-Transfers und aller Evolutionsperioden in einer geeigneten HF-Impulssequenz eliminiert werden.

6. Verfahren nach einem der vorhergehenden Ansprüche, dadurch gekennzeichnet, daß die Sequenz von HF-Impulsen einen Teil umfaßt, welcher dazu ausgebildet ist, NMR-Signale eines Lösungsmittels zu unterdrücken.

7. Verfahren nach Anspruch 6, dadurch gekennzeichnet, daß die Sequenz von HF-Impulsen einen Teil aufweist, welcher dazu ausgebildet ist, NMR-Signale von Wasser zu unterdrücken, insbesondere eine sogenannte "WATERGATE"-Nebensequenz mit zwei Off-Resonanz-HF-Impulsen am Ende der Sequenz, kurz vor der Datenerfassung.

8. Verfahren nach einem der vorhergehenden Ansprüche, dadurch gekennzeichnet, daß die verbleibende Linienbreite beinahe komplett auf die DD-Wechselwirkungen mit fernen Wasserstoffatomen in einem Protein zurückzuführen ist und daß die DD-Wechselwirkungen durch $^2$H-Markierung reduziert werden.

9. Verfahren nach einem der vorhergehenden Ansprüche, dadurch gekennzeichnet, daß das erhaltene heteronukleare Korrelationsspektrum zum Übertragen auf ein zweites Experiment verwendet wird, insbesondere ein NOESY-Experiment, welches Amid-Protonen und aromatische Protonen korreliert.

## Revendications

1. Procédé pour obtenir un spectre de corrélation de résonance magnétique nucléaire (RMN) de systèmes de spins hétéronucléaires, en particulier comprenant de grandes molécules, en particulier des macromolécules biologiques en solution, le système de spins étant soumis à un champ magnétique homogène $B_0$, irradié par une succession d'impulsions radiofréquence (rf) et comprenant au moins deux types de noyaux I et S de spin 1/2 couplés entre eux, caractérisé en ce que la succession d'impulsions rf est choisie de manière que l'élargissement de raies dans le spectre observé dû à la relaxation transversale ($T_2$) soit sensiblement réduit du fait d'une corrélation croisée entre le couplage dipôle-dipôle (DD) des spins et l'anisotropie des déplacements chimiques (CSA), en produisant deux vitesses de relaxation différentes des composants multiplets individuels du système de spins, de sorte que seuls sont enregistrés les signaux RMN du composant multiplet pour qui les contributions à la vitesse de relaxation transversale provenant d'interactions DD et CSA ont des signes différents.

2. Procédé selon la revendication 1, caractérisé en ce que la succession d'impulsions rf comprend une première impulsion à 90° non sélective affectant le premier type de noyaux I de spin 1/2, suivie par un temps de retard $\tau_1$, deux impulsions à 180° non sélectives affectant les noyaux I et S de spin 1/2, respectivement, suivies par un autre temps de retard $\tau_1$, une seconde impulsion à 90° non sélective. affectant le premier type de noyaux I, une première impulsion à 90° à cycle de phase $\psi_1$ agissant sur le second type de noyaux S, suivie par un temps d'évolution $t_1$, une première impulsion à 90° à cycle de phase $\varphi_1$ agissant sur le premier type de noyaux I, suivie par un temps de retard $\tau_1$, deux impulsions à 180° non sélectives affectant les deux types de noyaux I et S, respectivement, suivies par un autre temps de retard $\tau_1$, deux impulsions à 90° non sélectives affectant les noyaux I et S, respectivement, suivies par un temps de retard $\tau_1$, deux impulsions à 180° non sélectives affectant les noyaux I et S, respectivement, suivies par un temps de retard $\tau_1$ et une seconde impulsion à cycle de phase $\psi_2$ agissant sur le second type de noyaux S, suivie par une acquisition de données pendant une période de temps $t_2$.

3. Procédé selon la revendication 2, caractérisé en ce que des gradients de champ magnétique $G_1$, $G_3$, $G_4$ d'intensité différente sont appliqués au moins pendant des parties des intervalles de temps de retard $\tau_1$ et en ce qu'un gradient de champ magnétique $G_2$ est appliqué juste avant l'irradiation de la première impulsion à cycle de phase $\psi_1$ agissant sur le second type de noyaux S.

4. Procédé selon la revendication 2 ou 3, caractérisé en ce qu'aucune action de refocalisation n'est accomplie en ce qui concerne les systèmes de spins I, S pendant l'intervalle de temps d'évolution $t_1$ et la période de temps d'acquisition $t_2$, ce qui évite que les différentes vitesses de relaxation soient moyennées et l'élimination de l'interférence DD et CSA pendant ces périodes.

5. Procédé selon la revendication 4, caractérisé en ce que les impulsions rf de refocalisation à 180° pendant le transfert INEPT et toutes les périodes d'évolution sont éliminées dans une succession d'impulsions rf appropriée.

6. Procédé selon l'une des revendications précédentes, caractérisé en ce que la succession d'impulsions rf comprend une partie conçue pour supprimer les signaux RMN d'un solvant.

**7.** Procédé selon la revendication 6, caractérisé en ce que la succession d'impulsions rf comporte une partie conçue pour supprimer les signaux RMN de l'eau, en particulier une sous-succession appelée "WATERGATE" comprenant deux impulsions rf hors résonance à la fin de la succession juste avant l'acquisition des données.

**8.** Procédé selon l'une des revendications précédentes, caractérisé en ce que la largeur de raies résiduelle est due sensiblement totalement à des interactions DD avec des atomes d'hydrogène éloignés dans une protéine et en ce que les interactions DD sont réduites par marquage avec $^2H$.

**9.** Procédé selon l'une des revendications précédentes, caractérisé en ce que le spectre de corrélation hétéronucléaire obtenu est utilisé pour le relais par une seconde expérience, en particulier par une expérience NOESY qui corrèle les protons amides et les protons aromatiques.

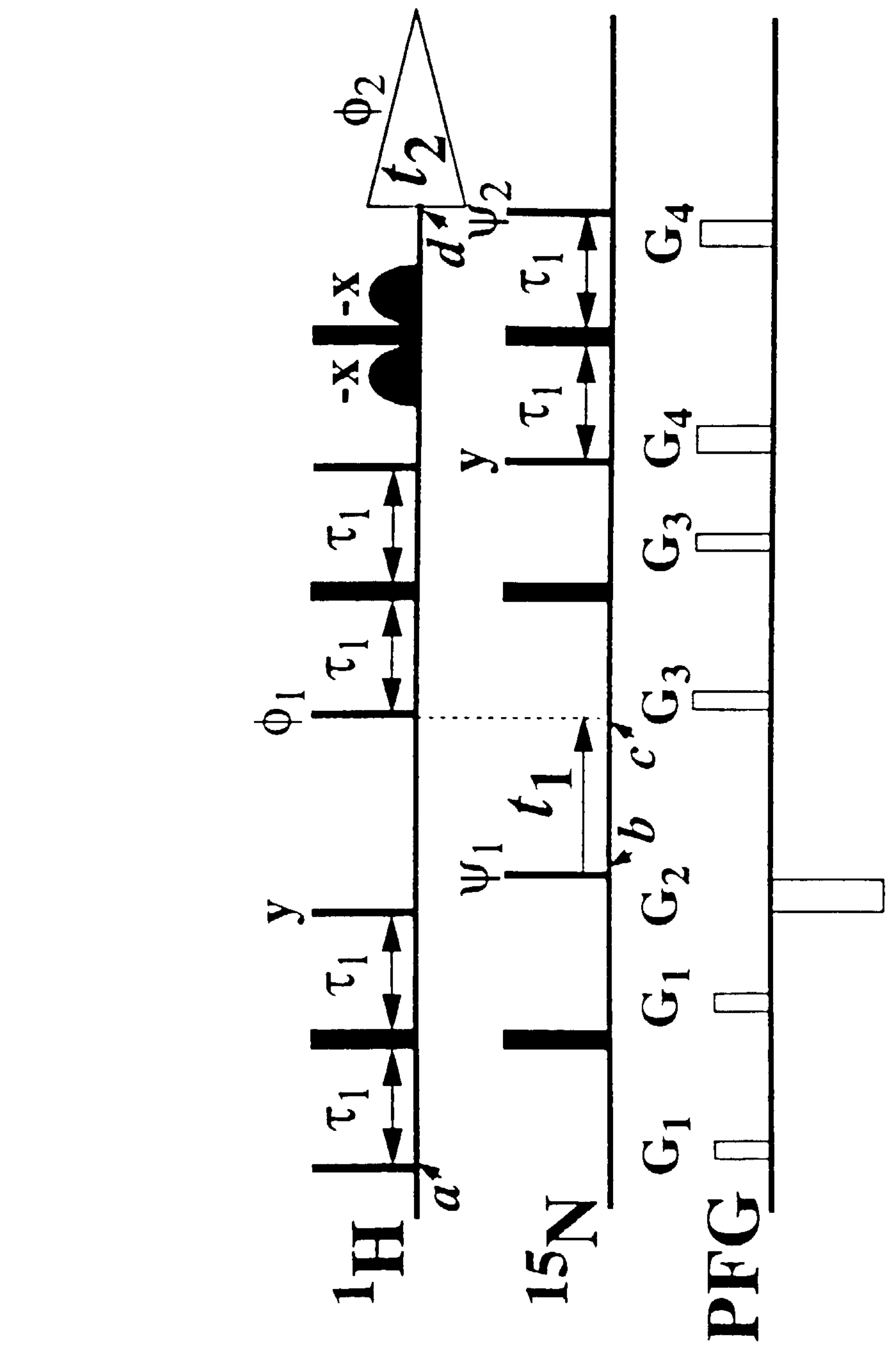


Fig. 1

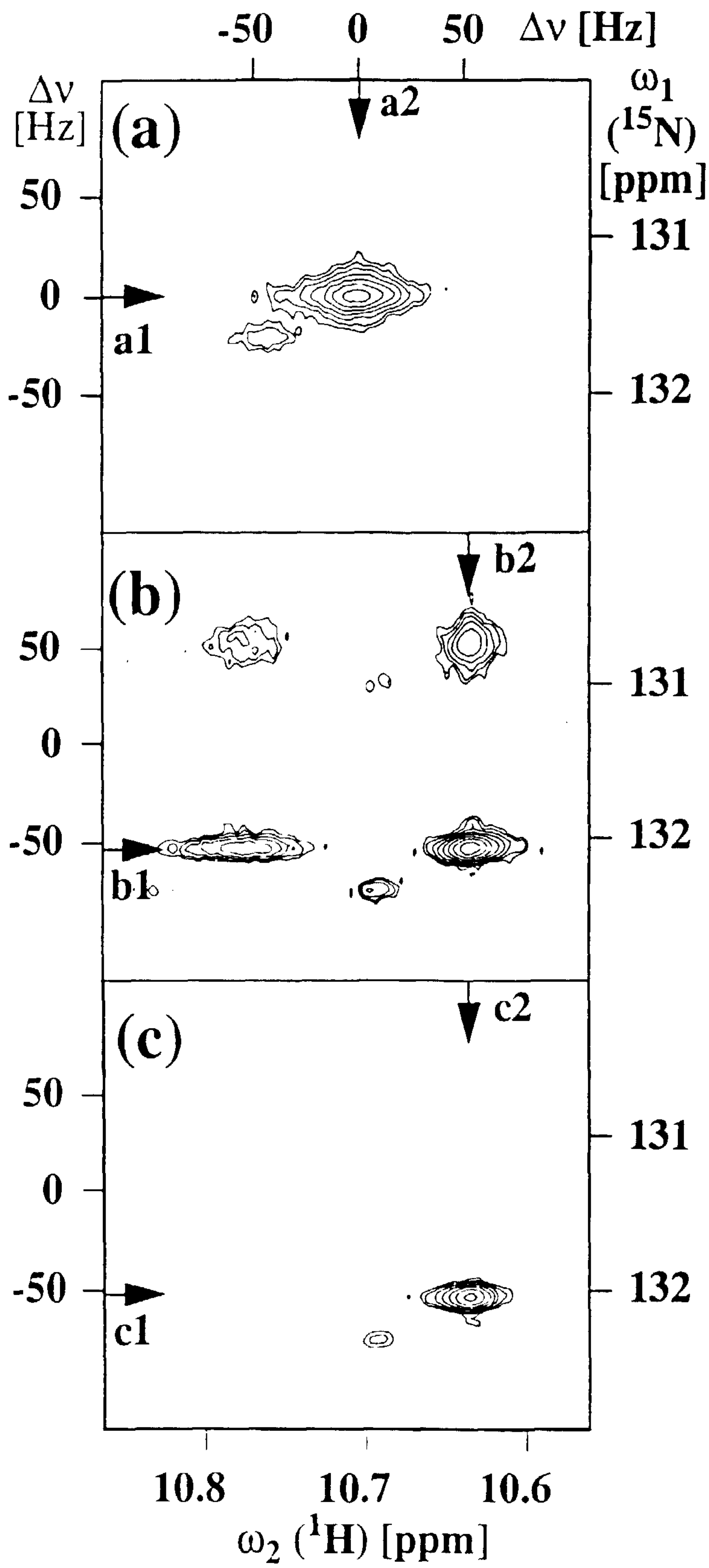
-50
0
50
Δν [Hz]
Δν [Hz]
ω1 (15N) [ppm]
(a)
a2
50
0
-50
a1
131
132
(b)
b2
50
0
-50
b1
131
132
(c)
c2
50
0
-50
c1
131
132
10.8
10.7
10.6
ω2 (1H) [ppm]

Fig. 2

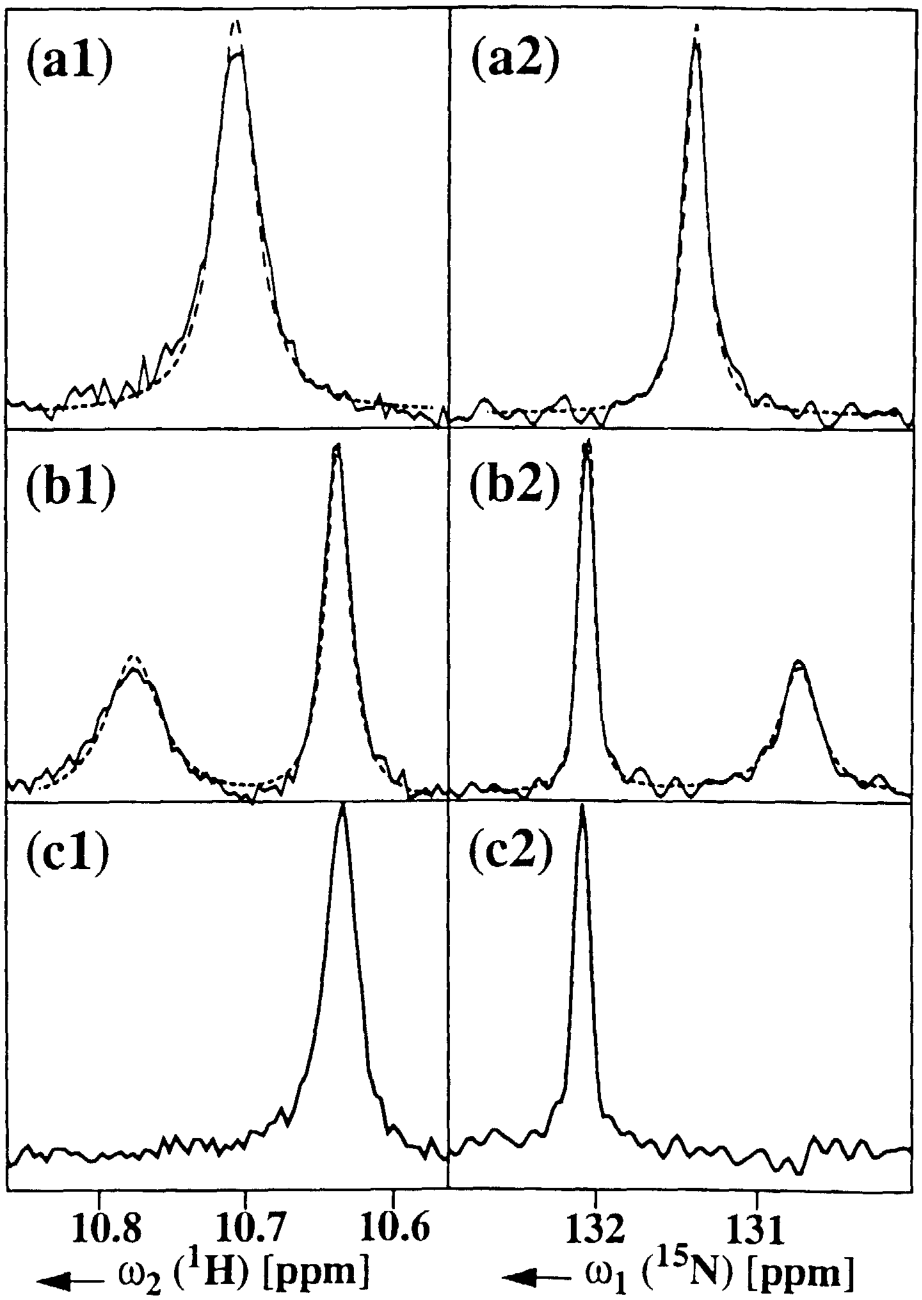


Fig. 3

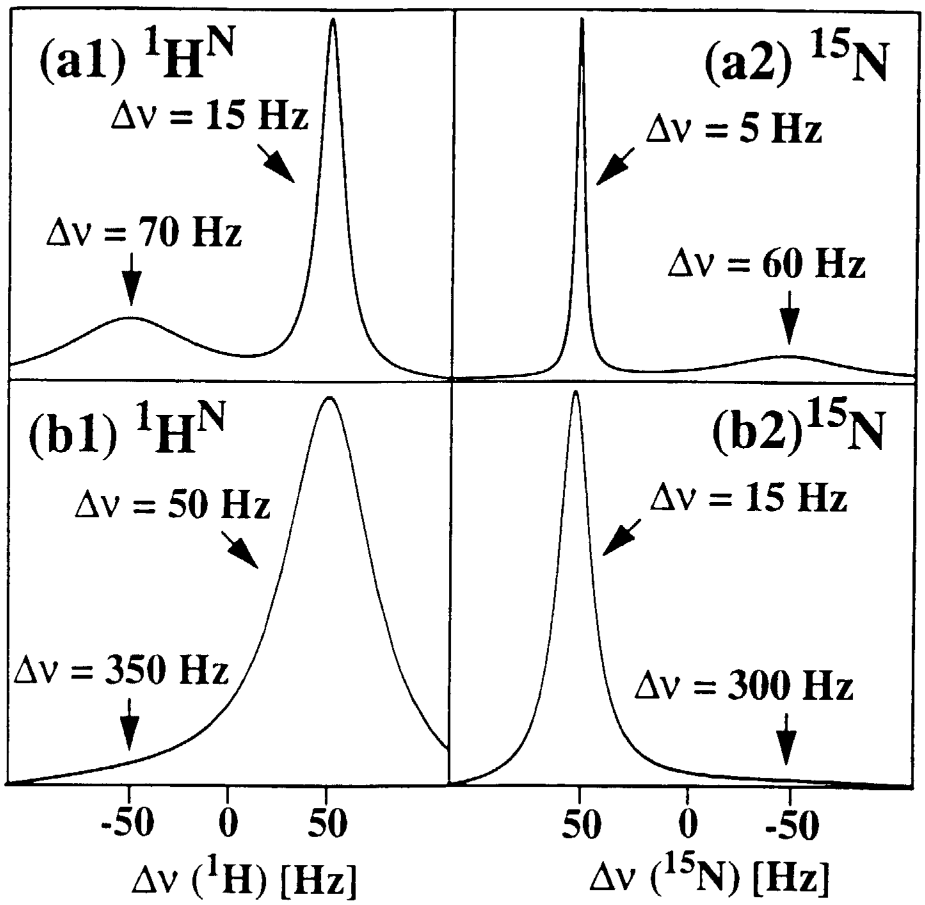
(a1) ¹H^N
Δν = 15 Hz
Δν = 70 Hz
(a2) ¹⁵N
Δν = 5 Hz
Δν = 60 Hz
(b1) ¹H^N
Δν = 50 Hz
Δν = 350 Hz
(b2) ¹⁵N
Δν = 15 Hz
Δν = 300 Hz
-50
0
50
Δν (¹H) [Hz]
50
0
-50
Δν (¹⁵N) [Hz]

Fig. 4